Europäisches Patentamt

**(19)** European Patent Office

Office européen des brevets

**(11)** Publication number: **0 146 847**
**A2**

**(12)** # EUROPEAN PATENT APPLICATION

**(21)** Application number: 84114831.5

**(22)** Date of filing: 06.12.84

**(51)** Int. Cl.⁴: **A 61 K 31/60**
**A 61 K 47/00**

**(30)** Priority: 13.12.83 IT 2414183

**(43)** Date of publication of application:
03.07.85 Bulletin 85/27

**(84)** Designated Contracting States:
AT BE CH DE FR LI SE

**(71)** Applicant: Di Bella, Agatina
Via Milano, 10
I-98023 Furci Siculo Messina(IT)

**(71)** Applicant: Macri di Bella, Giovanni
Via Milano, 10
I-98023 Furci Siculo Messina(IT)

**(71)** Applicant: Ferrara, Leonarda
Via Milano, 10
I-98023 Furci Siculo Messina(IT)

**(71)** Applicant: Adnet, Otello
Via Val Bavona 2
I-20147 Milano(IT)

**(71)** Applicant: Guastella, Giuseppe
Via Val Bavona 3
I-20147 Milano(IT)

**(72)** Inventor: Di Bella, Agatina
Via Milano 10
I-98023 Furci Siculo (Messina)(IT)

**(74)** Representative: Modiano, Guido et al,
MODIANO, JOSIF, PISANTY & STAUB Modiano &
Associati Via Meravigli, 16
I-20123 Milan(IT)

**(54)** Preparation for treating burns.

**(57)** The invention relates to a preparation for treating burns
of various seriousness and nature.
The preparation comprises a mixture of bulky salicylic
acid and American white flowable vaseline, which are cold
blended together.

EP 0 146 847 A2

Croydon Printing Company Ltd.

"PREPARATION FOR TREATING BURNS"

This invention relates to a preparation for treating burns of various seriousness and nature.

As is known, long available have been commercial products for treating burns originated by such various factors as the contact of epithelial tissue with high temperature elements or products, acids, or others.

These products are generally in the form of ointments or creams or dressings , so as to be easily applied onto a burned part, and are sometimes used, in the instance of extended and/or deep burns of some seriousness, concurrently with antibiotic coverings which are needed to prevent skin and/or widespread infections.

Such burn-fighting products are usually uncapable of regenerating tissue affected by the destructive process of a burn, and do not allow regeneration of the skin in a manner nearly overlapping the situation which existed prior to the noxa nociva, and do not prevent the occurrence of keloids  which are a fairly common incident with extended grade burns.

Almost always, moreover, the burned person, after application of other products to the burned tissue, does not feel the slightest relief owing to the substances contained in the product seldom providing an analgesic effect.

Application of commercially available products for  treating burns require freequent utilization over quite long time periods,before complete and perfect healing  of the burned part can be noticed, thus favoring the formation of keloids   at the location where the skin cells have been destroyed.

It is the aim of this invention to eliminate such prior shortcomings by providing a preparation for treating burns, which can ensure regeneration by new cells within a short time of that portion of the skin in which the   cells have been destroyed by the burn.

Within the above aim, it is an important object of the invention to provide a preparation for treating burns, which is effective to promote complete healing even of burn   types   of   high severity and extent.

A not unimportant object of the invention is to provide a preparation for treating burns which, additionally to having a healing effect, has an analgesic effect which soothes the patients' suffering.

This aim, as well as these and other objects, are achieved by a preparation for treating burns, characterized in that it comprises a mixture of salicylic acid and flowable or thready vaseline.

Further features and advantages of the invention will be more clearly apparent from the following description of a preferred, though not exclusive, embodiment of the preparation according to the invention. A preparation containing bulky or voluminous salicylic acid in an optimum proportion of 4% by weight is obtained by cold blending the salicylic acid with white preferably flowable vaseline of the American type in the amount of 96% by weight, with respect to the total weight of the preparation.

The percentages of salicylic acid and white vaseline may vary according to the seriousness of the burn to be treated; in particular, with minor burns, bulky salicylic acid in the amount of 2% blended with white American flowable vaseline in the amount of 98% may be used, all percentages being by weight.

The percentage of salicylic acid may be increased according to the severity and extent of the burn up to a maximum of 5.5% by weight, using therefore, an amount of vaseline corresponding to a percentage of 94.5% by weight.

The product comprising 4% salicylic acid and 96% white thready vaseline has been tested and used in 80 patient cases with burns in the first to third degree range and varying in extent and depth, obtaining within about 30 days complete and perfect recovery of 100% of the cases subjected to the treatment.

In those cases where the burn was of some seriousness, an antibiotic covering has also been used to prevent insurgence of infections; in all of the other cases, application of the product resulted, already within the first week, in regeneration of the skin cells affected by the burn.

In all cases, in the application of this preparation for treating burns, the preparation was liberally spread over the burn which was immediately bandaged with a sterile gauze whereon sanitary cotton was then laid for the purpose of absorbing harmful substances discharging from the burned skin. The sanitary cotton is then held in contact by an elastic bandage which allows the whole to be squeezed in position while permitting the skin to breathe.

The above-described procedure is repeated once every 24 hours without removing necrotic tissue before all of the degradation products have been eliminated, thereafter bandages are applied with the same procedure but at longer time intervals (every 48-76 hours etc.).

It has been ascertained in actual practice that the preparation for treating burns, according to the invention, is especially advantageous in preventing, during the application thereof, formation of keloids on the burned part.

The flowable or thready vaseline as preferably used in this invention was e.g. the white, soft, spreadable vaseline (soft paraphine) produced by the Italian Company ANGELINI FRANCESCO S.p.A. in Ancona (Italy) under the catalogue number 522026.

The voluminous Salicylic Acid as preferably used in this invention was the one produced by the Italian Company A.C.E.F. Azienda Chimica e Farmaceutica in Fiorenzuola d'Arda (Province of Piacenza) ITALY. Such salicylic acid is in the form of crystalline scales which are pulverized by rubbing them with a spatula before the amalgamation with vaseline. The term amalgamation as used herewith is intended to mean such admixing of the powder of salicylic acid into the vaseline at room temperature until a stable dispersion of the particles of salicylic acid within the vaseline is obtained.

### EXAMPLE

Burn in the first to second grade range to the face as caused by contact with boiling water. Damage extended to affect skin in the right forehead peri-orbital region - right zygomatic region - right cheek. The case came to our attention about four hours after the burning event. The patient in question had been subjected to no treatment. We operated as follows:

1) Application by means of a sterile spatula of a layer (2mm) of our invention (at 3,5 percent) over the entire burned part.

2) In contact with our invention sterile pads (gauze) have been applied so as not to overlap one another and closely contiguously over the entire burned area and neighbouring regions.

3) Over the sterile pads a sterile hydrophilic cotton layer has been applied to a thickness of about 1.5 mm.

4) Subsequently, a bandage was applied in a rather loose fashion using an elastic bandage so as not to compress the underlying burned tissue.

5) After approximately 24 hours the cotton layer was removed along with the sterile pad sheets. The cotton showed to have a layer from the inventive paste adherent thereto together with necrotic tissue portions. In fact, the main object of the cotton layer is that of absorbing the degraded burn produce which should never, in our method of treatment, be removed by any other techniques (wasting, wiping, or otherwise).

6) The said invention was freshly applied without washing, wiping, or else, as specified above.

Such procedures have been repeated every about 24 hours. In our experience, a progressive improvement was noticeable of the state of the burned part to complete healing within 20 days with complete re-epithelization of the skin layers and reconstruction of the affected tissue.

It is pointed out that during the last 5 days the burn has been treated with 2% preparation.

The invention as disclosed is susceptible to many modifications and changes without departing from the purview of the inventive concept; furthermore, all of the details may be replaced with equivalent elements.

0146847

## CLAIMS

1. A preparation for treating burns, characterized in that it comprises a mixture of salicylic acid and vaseline.

2. A preparation for treating burns, according to Claim 1, characterized in that said salicylic acid is provided in amounts varying from 2% to 5.5% and that said vaseline is a white preferably flowable vaseline in amounts varying from 98% to 94.5%, the percentages being by weight with respect to the total weight of the preparation.

3. A preparation for treating burns, according to Claims 1 and 2, characterized in that said salicylic acid is provided in an amount of 4% and said white flowable vaseline in an amount of 96%.

4. A preparation for treating burns, according to one or more of the preceding claims, characterized in that said salicylic acid is of the bulky type and said white flowable vaseline is of the American type.

5. A preparation for treating burns, according to one or more of the preceding claims, characterized in that said salicylic acid and said white flowable vaseline are cold blended together.

6. Method for treating burns, characterized in that it comprises applying the preparation of Claims 1-5 on the burn to be treated for a selected time period.